# EUROPEAN PATENT APPLICATION

(11) **EP 2 006 354 A2**
(43) Date of publication of application: **24.12.2008**
(21) Application number: 06742017.4
(22) Date of filing: 29.05.2006
(51) Int. Cl.: C10G 1/06, C12P 7/06, B09B 3/00, C12P 1/00, B27K 9/00

(54) **NOVEL METHOD FOR PRODUCTION LIQUID FUEL FROM BIOMASS**

(30) Priority: 27.02.2006 CN 200610008062
(71) Applicant: Pficker Pharmaceuticals Ltd., 235000 Anhui (CN)
(72) Inventor: YIE, Hongping, Anhui 235000 (CN); SUN, Meg M., San Diego, CA 92128 (US); ZHU, Zuolin, San Diego, CA 92128 (US); CHEN, Pei, San Diego, CA 92128 (US); LI, Yueping, Beijing 100028 (CN); TAUBE, William, San Diego, CA 92128 (US); ZHU, Zuodong, Anhui 235000 (CN); GONG, Jiangen, San Diego, CA 92128 (US); WU, Jason, San Diego, CA 92128 (US)
(74) Representative: Bottero, Carlo
(86) International application number: PCT/CN2006/001129
(87) International publication number: WO 2007/095787

(57) **Abstract**

The present invention provides a novel process of producing liquid fuel from biomass, wherein the pretreatment is performed at ambient temperature and pressure through use of force field and catalyst. The process is carried out in a mild condition and easy to be operated. Moreover, the process will not generate substances detrimental to hydrolysis and fermentation. Production cost is thereby reduced and the biomass pretreatment effect and the extraction degree of lignin is increased. The process can more effectively and completely utilize biomass, and additionally, it can be used in combination with various post-processing methods of biomass.

## Description

### TECHNICAL FIELD

The present invention relates to a novel method of producing liquid fuel through refining of biomass. In particular, the present invention relates to a method of producing gasoline by pretreatment of biomass at ambient temperature and pressure and collection of lignin.

### BACKGROUND ART

Fossil resource, which is not renewable, such as, coal, petroleum, nuclear energy, and natural gas, are consumed more and more rapidly with the progress of the science and technology and the rapid development of global industrialization. The consumption of fossil resource has reached or is stepping into its maximal amount that is available. In order to ensure the living and sustainable development of human being, we need to develop and utilize new, renewable energy resource.

In comparison with resource that is not renewable, renewable resource, such as wind energy, solar energy, and biomass energy, etc, will not diminish by consumption and has the least impact on environment. Among these resources, biomass energy is energy based on biomass. Biomass is all the living organic matter that can grow, including animal, plant, and microorganisms. Biomass is converted from solar energy. Green plant, algae, and photosynthetic bacteria on the earth store chemical energy through photosynthesis. Biomass energy has vey large potential. There are about 250 thousand of organisms on the earth, which, in an ideal environmental condition, has the highest photosynthesis efficiency of up to 8-15%, with generally an average of 0.5%. It is estimated that the biomass energy fixed by photosynthesis every year over the world is 10 times the overall annual energy consumption by the world. This form of energy is transformed into carbon dioxide and water during its use, and renewed again by photosynthesis.

Development and utilization of technologies efficiently converting biomass-energy-enriched materials into liquid fuel such as gasoline, diesel oil, ethanol, etc, can ensure living and sustainable development of human beings.

There are two major classes of process utilizing biomass to producing ethanol as liquid fuel. One process produces ethanol by directly hydrolyzing biomass with acid to give monosaccharide and fermenting the isolated monosaccharide to give ethanol product; the other process initially produces monosaccharide and lignin by bio-transformation method.

Acid hydrolysis takes a long time and produces by-products detrimental to fermentation. If concentrated acid is used, an expensive and complex process for recovering the acid is required. Whereas, hydrolysis with diluted acid needs high temperature (above 200 °C) and high pressure, and furthermore produces by-products detrimental to fermentation. The by-products generated in hydrolysis and detrimental to fermentation must be removed before the fermentation begins. Cost of this process is high and difficult to be decreased due to complexity of the process.

Enzymatic hydrolysis converts polysaccharide in biomass into monosaccharide useful in fermentation with catalytic enzymes produced by microorganisms. Enzymatic hydrolysis has a number of advantages including that it can be performed at ambient or a slightly higher temperature; culture and maintenance of microorganisms only need a small amount of raw materials; energy expenditure of the process is low; enzyme may have high selectivity and produce a single product, thereby giving a very high yield of monosaccharide (>95%); there are substantially no chemicals included in enzymatic hydrolysis and only a minimum amount of by-products are produced, thus this process does not impose environmental pollution and the purification process is comparatively simple.

However, hydrolysis catalyzed by enzyme is extremely slow unless the physical and chemical hindrances for the contact of enzymes and cellulose can be overcome. The physical hindrance includes the tighten crystal structure, very small surface area, high polymerization degree difficult to be degraded of the biomass; the chemical hindrance includes lignin, hemicellulose, and acetyl group shielding the cellulose. These physical and chemical hindrances play an interacting role during the enzymatic hydrolysis process. Hydrolysis of biomass using enzymes alone suffers from the prolonged time period, and biomass needs to be effectively pretreated to get effectively hydrolyzed. Pretreatment can remove lignin, dissolve hemicellulose and disrupt the crystal structure of cellulose, thereby increasing the accessible surface of the saccharide polymer in the biomass and improving hydrolysis rate.

All the available technologies of biomass pretreatment have their limitations. For example, the pure physical process suffers from large energy consumption and poor effect; the pure chemical method suffers from a long reaction time, high cost, and a large quantities of waste water; the pure biological method is too slow; the steam explosion method (a physical and chemical method), which needs high temperature, high pressure and special equipment, produces substances detrimental to fermentation, poor effect and large loss of xylose; the ammonia fiber explosion method (also a physical and chemical method) also needs high temperature, high pressure and special equipment, and the decomposition of hemicellulose needs additional steps; CO₂ explosion method (also a physical and chemical method) also needs high temperature, high pressure and special equipment but produces fairly poor effect. A good pretreatment process shall meet all the following criteria:
①the process can promote the formation of monosaccharide or facilitate the subsequent enzymatic hydrolysis;
②the process avoids the lose of carbohydrates due to degradation;
the process does not generate by-products detrimental to hydrolysis and fermentation;
④the process is economically reasonable.

### CONTENT OF THE INVENTION

The objective of the present invention is to provide a novel method of producing liquid fuel from biomass, wherein the pretreatment of biomass is carried out at ambient temperature and pressure, does not produce substances detrimental to hydrolysis and fermentation, has decreased degradation loss of carbohydrates in the biomass, minimizes the amount of water used in the treatment, and favorites the subsequent enzymatic hydrolysis.

Another objective of the present invention is to provide a novel method for more effectively and completely utilizing biomass, improving the added value of by-products, thereby making the use of biomass more economically reasonable.

According to the first aspect, the present invention provides a method for the pretreatment of biomass, comprising the steps of:
a) Providing milled biomass;
b) Mixing the biomass obtained in step a) and the 0.001wt%-50wt% aqueous solution of catalyst to make a mixture of the biomass and catalyst, wherein the weight versus volume ratio of the biomass and the aqueous solution of catalyst is 1:1-1000;
   Wherein said catalyst is selected from a water-soluble alkali compound or a water-soluble diluted acid compound;
c) treating the mixture obtained in step b) by using a force field at ambient temperature and under ambient pressure for a time period ranging from 1 second to 1000 minutes, then isolating the treated mixture by filtration to give the pretreatment product of the biomass, wherein said pretreatment product includes liquid and solid portions.

In a preferred example, the water soluble alkali compound is selected from the group consisting of aqueous ammonia, lithium hydroxide, sodium hydroxide, potassium hydroxide, calcium hydroxide, sodium carbonate, potassium carbonate, calcium oxide, or the combination thereof; the water soluble diluted acid compound is selected from the group consisting of diluted hydrochloric acid, diluted sulfuric acid, diluted phosphoric acid, or the combination thereof.

According to another aspect, the present invention provides a method of producing liquid fuel from biomass, including the steps of:
(i) Hydrolyzing the solid portion of the pretreatment product with enzymatic catalyst to obtain monosaccharide, which, in turn, is fermented and purified to give the first liquid fuel;
(ii) isolating the liquid portion of the pretreatment product to obtain lignin, which, in turn, is hydrogenized to give the second liquid fuel.

The first liquid fuel includes but not limited to ethanol. The second liquid fuel includes but not limited to gasoline.

In a preferred example, the solid portion obtained in step c) is washed with water or hot steam in step (i), then hydrolyzed with enzyme into monosaccharide, fermented to produce ethanol solution, and distilled and fractionated to obtain the ethanol fuel.

In a preferred example, in step (ii) the liquid portion is extracted with polar aromatic compound to isolate lignin, which is directly used as raw material for the production of gasoline through hydrogenation.

In another preferred example, in step (ii) lignin is obtained by evaporating the liquid, then the lignin is directly used as raw material to produce gasoline through hydrogenation.

In a preferred example, the catalyst is aqueous ammonia of 0.001wt%∼30wt%, and ratio of the biomass and the aqueous ammonia is 1:1∼1000 (weight/volume).

In a preferred example, the aqueous ammonia has a concentration of 5wt% to 25wt%, more preferably, 10wt% to 20wt%.

In a preferred example, ratio of the biomass and the aqueous ammonia is 1:2 ∼20(weight/volume), more preferably, 1:3 ∼ 10.

In a preferred example, the force field is microwave, ultrasound, or the combination thereof.

In a preferred example, frequency of the microwave is 300 megahertz (MHz) to 300 gigahertz (GHz), intensity of the microwave is between the range from 0.1 W to 10kW.

In a preferred example, intensity of the microwave is preferably between the range from 100 to 3000 W.

In a preferred example, the frequency of the microwave is between the range from 17kHz to 300MHz, more preferably, 18kHz to 100MHz; intensity is between the range from 0.1 Watts/cm² to 300 Watts/cm², more preferably, 40Watts/cm² to 100 Watts/cm².

In a preferred example, the duration time of step c) is between the range from 1 minute to 60 minutes, more preferably, 5 to 30 minutes.

In a preferred example, in step a) the biomass is incubated with the lime of 1-100wt% for a time between the range from 1 to 180 days.

In a preferred example, the method of isolating the liquid product in step (ii) is extracting the liquid product with polar aromatic compound, said polar aromatic compound is selected from the group consisting of mono-substituted benzene, poly-substituted benzene, aqueous naphthalene derivative, aqueous phenanthrene derivative, aqueous anthracene derivative, or the combination thereof.

In a preferred example, the diluted aqueous ammonia collected from washing of the solid product with water or hot steam in step (i) and the diluted aqueous ammonia left after extraction of lignin from the liquid product in step (ii), upon adjustment of the concentration by addition of concentrated aqueous ammonia, is used in step b).

In a preferred example, the process for hydrogenation is the melted zinc chloride method.

According to still another aspect, the present invention provides use of biomass, that is, use of biomass for the simultaneous production of ethanol liquid fuel and gasoline liquid fuel.

### BRIEF DESCRIPTION OF THE DRAWINGS

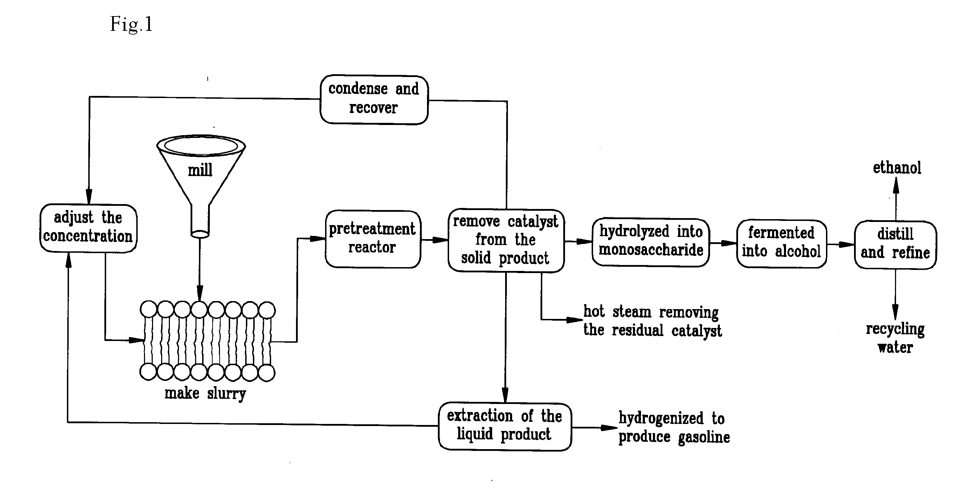

The figure is a schematic flow chart of ethanol fuel production, wherein the residual aqueous ammonia is removed by hot steam.

### BEST MODES FOR CARRYING OUT THE INVENTION

Inventors of the present invention, through extensive and intensive investigations, have obtained a highly efficient and low energy-consuming method for producing liquid fuel from biomass by providing the invented process. Moreover, the inventors has surprisingly found that biomass can be rapidly pretreated under a mild condition, namely, the ambient temperature and pressure, by the synergetic effect of the catalyst and force field according to the present invention. The inventors accomplished the present invention on this basis.

The present invention is now described in more detail:

### Biomass

As used herein, "biomass" refers to the substances rich in biomass energy left after removal of eatable portion, for example, various grass and trees (including various water plant), urban wood waste, stem, leaf, and root of crops, nutshell, wood waste of paper mill, sawdust, offal of oil or sugar press, and scrap of forest cutting, etc.

Biomass in step (a), when stored, may be placed together with lime (e.g. white lime), which will facilitate biomass pretreatment.

Biomass according to the present invention may be milled biomass solid, including but not limited to, biomass having a particle size in the range between 0.01mm to 8mm, preferably 0.1mm to 5mm, and content of water between 1wt% to 50wt%, preferably 5wt% to 30wt%, calculated on the basis of the total weight of said biomass.

### Force field

Said force field is microwave, ultrasound, or concurrent ultrasound - microwave. The force field employed in the present invention, which can accelerate the biomass pretreatment reactions, include microwave, ultrasound or ultrasound-microwave in a synergetic manner. Microwave electromagnetic field can change the polar molecules in biomass from the initial thermal motion to be oriented according to the alternating orientation of the electromagnetic field. The crystal structure of biomass is disrupted by the energy of microwave alternating electromagnetic field or cavitations induced by ultrasonic vibration, resulting in the decreased polymerization degree of the biomass, disruption of the covalent bond between lignin and polysaccharide in the biomass, and hydrolysis of the most acetyl group in the biomass.

Frequency of the microwave used in the present invention is between 300 megahertz (MHz) to 300 gigahertz (GHz, wavelength between 1 meter to 1 millimeter), and strength of the microwave is generally between 0.1W to 10kW, commonly between 100 to 3000W. If the frequency exceeds 300 gigahertz or the strength exceeds 10kW, energy expenditure increases greatly, though the effect of biomass treatment is slightly improved; if the frequency is less than 300 megahertz or the strength is less than 0.1 W, the treatment effect is poor, although the energy expenditure is decreased. Microwave in the range disclosed herein, preferably 1000W/2.45GHz, will produce excellent effect of pretreatment.

Frequency of ultrasound used in the present invention is between the range of 17kHz to 300MHz, most preferably between 18kHz to 100MHz, and strength of the ultrasound is between the range of 0.1W to 10 kW (0.1 Watts/cm² to 300 Watts/cm²), most preferably between 1kW to 6 kW (40Watts/cm² to 100 Watts/cm²). If the frequency exceeds 300MHz or the strength exceeds 10kW, energy expenditure increases greatly, though the effect of biomass treatment is slightly improved; if the frequency is less than 17kHz or the strength is less than 0.1W, the treatment effect is poor, although the energy expenditure is decreased. Ultrasound in the range disclosed herein, preferably 2kW/20kHz, will produce excellent effect of pretreatment.

Ultrasound can be generated by mechanic, electric, electromagnetic, or heat energy method. A typical electromagnetic source is a magnetostrictive transducer, which transforms magnetic energy into ultrasonic energy by applying alternating magnetic field to some specific metal, alloy, or ferrite. A typical electric source is a piezoelectric transducer which transforms electric energy into ultrasonic energy by applying alternating voltage on the front face of monocrystal (natural or synthetic, for example, quartz) or ceramic (barium titanate ceramic or lead zirconate ceramic) to induce alternating expansion and contraction of crystal or ceramic under the exogenously applied frequency.

According to the present invention, microwave and ultrasound may act on the reaction system for 1 second to 1000 minutes, generally between 1 minute to 60 minutes, preferably 5 minutes to 30 minutes.

### Catalyst

The catalyst used in the present invention includes water soluble alkali compound, such as aqueous ammonia, lithium hydroxide, sodium hydroxide, potassium hydroxide, calcium hydroxide, sodium carbonate, potassium carbonate, and calcium oxide, etc; and water soluble diluted acid compound, such as diluted hydrochloric acid, diluted sulfuric acid, and diluted phosphoric acid, etc. The ratio of biomass (dry weight) and catalyst (weight/volume, or solid/liquid) can be in the range of 1:1-1000, concentration of the catalyst solution in water is between the range of 0.001wt%-50wt%.

In the case that the catalyst is aqueous ammonia, the diluted aqueous ammonia collected from flushing of the solid product or from condensing of the hot steam after washing the solid product and the diluted aqueous ammonia left after extraction of lignin from the liquid product in step (ii), upon adjustment of the concentration by addition of concentrated aqueous ammonia, can be used in step b).

To facilitate recovery, aqueous ammonia is preferable in the present invention. Concentration of the aqueous ammonia in weight percentage can be in the range of 0.001wt% to 30wt%, generally 5wt% to 25wt%, most preferably 10wt% to 20wt%. If concentration of the aqueous ammonia is greater than 30wt%, the treatment effect is not better than that of the concentration of 15wt%, while the cost noticeably increases; if concentration of the aqueous ammonia is less than 0.001wt%, then the treatment time is too long to be economically acceptable.

The weight/volume (solid/liquid) ratio of biomass and aqueous ammonia is typically 1:2 -20, more preferably 1:3 -10. The solid/liquid ratio of 1:3 can produce wonderful pretreatment effect of biomass when a suitable reaction condition is also provided.

### Steps of biomass pretreatment

Biomass pretreatment according to the present invention comprises the following three steps:
a) Milling the dried biomass;
b) mixing the milled biomass and the catalyst in a ratio of 1:1∼1000 (w/v, solid/liquid), wherein the catalyst is in a water solution of 0.001wt%∼50wt%, catalyst used either be water soluble alkali compound, such as aqueous ammonia, lithium hydroxide, sodium hydroxide, potassium hydroxide, calcium hydroxide, sodium carbonate, potassium carbonate, and calcium oxide, etc; or water-soluble diluted acid, such as diluted hydrochloric acid, diluted sulfuric acid, and diluted phosphoric acid;
c) treating the mixture by using a force field at ambient temperature and ambient pressure for a time period ranging from 1 second to 1000 minutes, then isolating the treated mixture by filtration to give solid product and liquid product.

The already known processes for producing liquid fuel from biomass include direct cracking of biomass, such as rapid cracking, rapid hydrocracking, vacuum cracking, low temperature cracking, partial combustion cracking, etc; processes similar to coal gasification to produce syngas which is then converted into products such as methanol; and process to refine biomass to produce ethanol and gasoline. Process to produce ethanol and gasoline fuels makes the greatest conversion rate of biomass and hence is believed to be one of the major technologies for energy supply. The most essential factor influencing production cost of biomass pretreatment is the temperature and the total amount of liquid used in the biomass pretreatment reaction.

The biomass pretreatment process according to the present invention can be performed at the ambient temperature and under the ambient pressure, in comparison with those known biomass pretreatment methods that need high temperature and high pressure. The pretreatment reactor used in the present invention can be a reactor that is suitable for use at the ambient temperature and under the ambient pressure. A common pressure reactor is three times more expensive than a ordinary reactor; production cost of a pretreatment under ambient temperature and ambient pressure will be decreased compared with that under high temperature and high pressure; the amount of catalyst used is decreased, and moreover, the catalyst can be recycled, which can also reduce cost of the pretreatment; the mild operation condition reduces deterioration of the apparatus; the pretreatment reaction does not produce any substance detrimental to the subsequent enzymatic catalysis, thereby the present invention does not need additional detoxification steps and hence reducing operation cost of the overall manufacturing; this novel pretreatment process can recover higher than 90% lignin, thereby increasing added value of the product and hence further reduce the total cost of the entire process.

The biomass pretreatment process of the present invention can be performed under ambient temperature and under ambient pressure. As such, the invented method avoids un-reversible reaction between lignin and cellulose under high temperature and high pressure, the product of reaction is extremely difficult to be dissolved again. Therefore, the present invention improves the effect of biomass pretreatment and increases the degree of lignin extraction.

The method of biomass pretreatment according to the present invention can overcome the physical and chemical hindrance in hydrolysis of biomass by one-step mild reaction. This pretreatment disrupts crystal structure of biomass, increases surface areas of biomass, decreases polymerization degree of biomass, isolate lignin, and remove most acetyl group of biomass. Furthermore, hemicellulose in the biomass can be retained to the maximal extent, if desired.

In a preferred example, biomass is treated with a system of force field and aqueous ammonia. In the present invention, a system of microwave and aqueous ammonia gives results superior to that of a system of ultrasound and aqueous ammonia in the same time of reaction in terms of enzymatic hydrolysis degree of the biomass and removal degree of lignin from the biomass. However, the system of microwave and aqueous ammonia loses more xylan and temperature in the reaction system climbs too rapidly. These systems can be selected as needed.

Heat generated during the action of microwave and ultrasound on the reaction system does not affect the pretreatment effect of biomass.

According to the present invention, 95% of the cellulose in the pretreated biomass is retained as the solid portion, and 90% of the lignin can be removed from the biomass and retained in the liquid portion in appropriate conditions.

According to the present invention, the biomass can be stored together with lime. This will facilitate biomass pretreatment. Biomass can be stored for 1 to 180 days, with the amount of lime between 1% to 100% of the weight of the biomass material. Biomass treated in this way will then have better enzymatic hydrolysis degree and removal degree of lignin when compared with that without lime. Additionally, lime can compress the biomass, deform the lignin, enhance viscosity of the biomass to make it easier for compressing, and hence increases density and decreases volume of biomass, thereby facilitating the storage and transportation of them.

The pretreatment reaction can be carried out in a batch reactor system, or a continuous flow reactor system, or a flow through reactor system.

The biomass pretreatment reaction according to the present invention, which can occur in a mild condition, is easy to be operated, and can be combined with various downstream processes of biomass, for example, monosaccharide process, acetic acid process, alcohol process, and other processes producing liquid and solid products, etc.

According to the present invention, the pretreated biomass does not contain detrimental or toxic by-products affecting the enzymatic hydrolysis and monosaccharide fermentation, such as, aldehyde by-product, for example, Furfural, acid by-product, for example, acetic acid, and some metal ions, etc. In addition, the pretreated biomass does not contain other microorganisms due to the microbicidel effect of microwave, ultrasonic wave, or the combination of microwave and ultrasonic wave, and thus decreases the risk of microbial contamination in hydrolysis and monosaccharide fermentation.

### Production of liquid fuel using the pretreatment product of biomass

The biomass undergone pretreatment include two portions, namely, solid and liquid, wherein the solid portion is primarily cellulose, hemicellulose, and a minimum amount of lignin; the liquid portion is primarily lignin, hemicellulose, and a minimum amount of cellulose. The solid and liquid portions can be separated by conventional separation method.

The separated solid portion is used to produce the first liquid fuel, by using methods including but not limited to the following: removing the residual catalyst and lignin by rinsing with a little water. If aqueous ammonia is used as the catalyst, hot steam can be used to remove the residual aqueous ammonia, and then the solid product is directly used in enzymatic hydrolysis to give monosaccharide that can be fermented. The accompanying drawing shows the flow chart of the process of producing ethanol fuel, wherein the residual aqueous ammonia is removed with hot steam. The process using water rinsing does not have much difference with the process using hot steam. The difference only lies as the obtained solid product is rinsed with water and the eluant is pooled into the liquid product. The aqueous ammonia, after extraction of lignin from the liquid product, is recycled by being adjusted to an appropriate concentration by adding concentrated aqueous ammonia and then used for biomass pretreatment again. If the liquid product contains too much impurity after too many recycles, the liquid product, after being extracted for lignin, is eluted with hot steam to remove all the ammonia, and the liquid portion is no longer subjected to recycling, but the condensed aqueous ammonia goes to the recycle. Whereas hot steam is used to rinse solid product to remove ammonia, the residual aqueous ammonia is gone with the hot steam, and then condensed to be liquid and enters into the adjustment reactor, wherein it is adjusted to have a suitable concentration by adding concentrated aqueous ammonia, and then the aqueous ammonia is used again for biomass pretreatment-recycling.

Lignin and hemicellulose in the liquid portion is used to produce the second liquid fuel. The liquid product can be treated by various methods, including but not limited to the following: the first method is to extract the liquid portion with aromatic compounds, wherein aromatic compounds refer to all the aromatic compounds each of which has a single component, such as monosubstituted benzene, for example, toluene; polysubstituted benzene, for example, xylene; liquid naphtalin derivative, for example, tetrahydronaphthalene; liquid phenanthrene derivative, for example, dihydrophenanthrene; liquid anthracene derivative, for example, dihydroanthracene, tetrahydroanthracene; etc. What is extracted with the aromatic compound is almost solely lignin. The aqueous ammonia solution comprising hemicellulose after said extraction is adjusted to a desired ammonia concentration and used in biomass pretreatment recycling. As the second method, after lignin is extracted from the biomass with an aromatic compound, aqueous ammonia is removed from the aqueous ammonia solution comprising hemicellulose by using hot steam and as such the hemicellulose is isolated. The condensed aqueous ammonia is recovered and used again in biomass pretreatment. In the third method, the liquid portion comprises dominant lignin, hemicellulose, and a minimum amount of cellulose, after removal of ammonia by hot steam, is directly used to produce marsh gas (methane gas). The marsh gas can provide power to systemic processes, or can be used as fuel to directly provide heat to production. In the fourth method, the liquid portion comprises lignin as the dominant component, hemicellulose, and a minimum amount of cellulose, is directly dehydrated to dryness to give a solid product comprising lignin as the main component.

Lignin obtained by pretreatment of biomass can be subjected to a series of separation procedures to give various aromatic compounds, or it can be directly used in hydrogenation process to produce oil. All the known technologies for liquefying coal to produce oil can be used herein to convert lignin into gasoline, with the liquefaction process catalyzed by melted zinc chloride being the most preferable.

The following examples are provided to better illustrate present invention, and shall not be considered as limiting the invention. The biomass pretreatment process provided in the present invention can be used for all kinds of biomass. Separation of various major components are performed according to the methods disclosed in the specification, and applications of these components are those disclosed herein.

The biomass used in the following examples includes but does not limited to milled maize straw (i.e. straw), wheat straw, soybean straw and cotton straw that has been air dried. Particle size of the biomass is in the range of 1.6 to 2.4 mm. In an embodiment, a particle of maize straw consists of 36.4wt% β-glucan, 18.8wt% xylan, 2.8wt% arabinan, 1.8wt% mannan, 2.2wt% galactan, 20.2wt% Klason lignin, 7.0wt% ash, 3.2wt% acetyl group, 4.0wt% protein, and 3.8wt% Uronic acid; a particle of wheat straw consists of 33wt% β-glucan, 45wt% xylan, and 20wt% Klason lignin; a particle of soybean straw consists of 41wt% β-glucan, 23wt% xylan, and 20.8wt% Klason lignin; a particle of cotton straw consists of 41.4wt% β-glucan, 23.8wt% xylan, and 20.6wt% Klason lignin.

Enzymatic digestibility of cellulose biomass such as maize straw is measured according to NREL Chemical Analysis and Testing Task Laboratory Analytical Procedure, LAP-009.

Content of saccharide and lignin in the liquid sample: measured by HPLC after acid treatment according to NREL Chemical Analysis and Testing Standard Procedure, 001-004, 1996.

Yield of ethanol: measured by gas chromatography.

Hydrolysisability of the pretreated cellulose biomass such as maize straw: measure by using Spezyme CP cellulose hydrolase (Genencor, average activity being 30.6FPU/ml and 20CBU/ml, protein of 106 mg/ml). Activity of the β-glucosidase (Novozyme) employed in this assay is 5.0CBU/mg. Saccharomyces *cerevisiae* NREL-D₅A is used in Simultaneous Saccharification and Fermentation, with the growth media YP (peptone agar medium) comprising 1% of yeast extract (Sigma) and 2% of peptone (Sigma).

### Example 1: influence of pretreatment time with ultrasound on the pretreatment effect

20 g of air dried, milled maize straw were added into a suitable container, and then 300 ml aqueous ammonia of 15wt% were added as solvent. The container was closed and sealed appropriately and then treated by ultrasound in a 2kW/20kHz ultrasound reactor with the container comprising the maize straw continually shaken during the pretreatment. Sample obtained in this way was Sample 1-2. Samples 1-3, 1-4 and 1-1 were used in comparative experiments. Sample 1-3 was prepared by using deionized water as solvent and pretreatment by a 2kW/20kHz ultrasound for 60 minutes; sample 1-4 was prepared by using aqueous ammonia as solvent and no ultrasound treatment; sample 1-1 was not subjected to any treatment. After treatment, about 10ml liquid sample was taken from the container comprising the maize straw and used for analysis, the other liquid sample treated by ultrasound was pooled together and extracted with toluene (2 × 400 ml). The solid sample was washed with deionized water to remove aqueous ammonia, dried under deep freeze and vacuum, and then sampled for solid composition analysis and enzymatic digestibility analysis. The results are shown in Table 1.

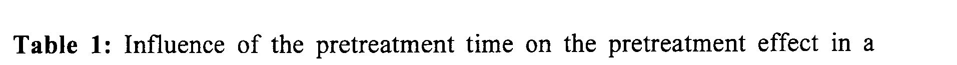

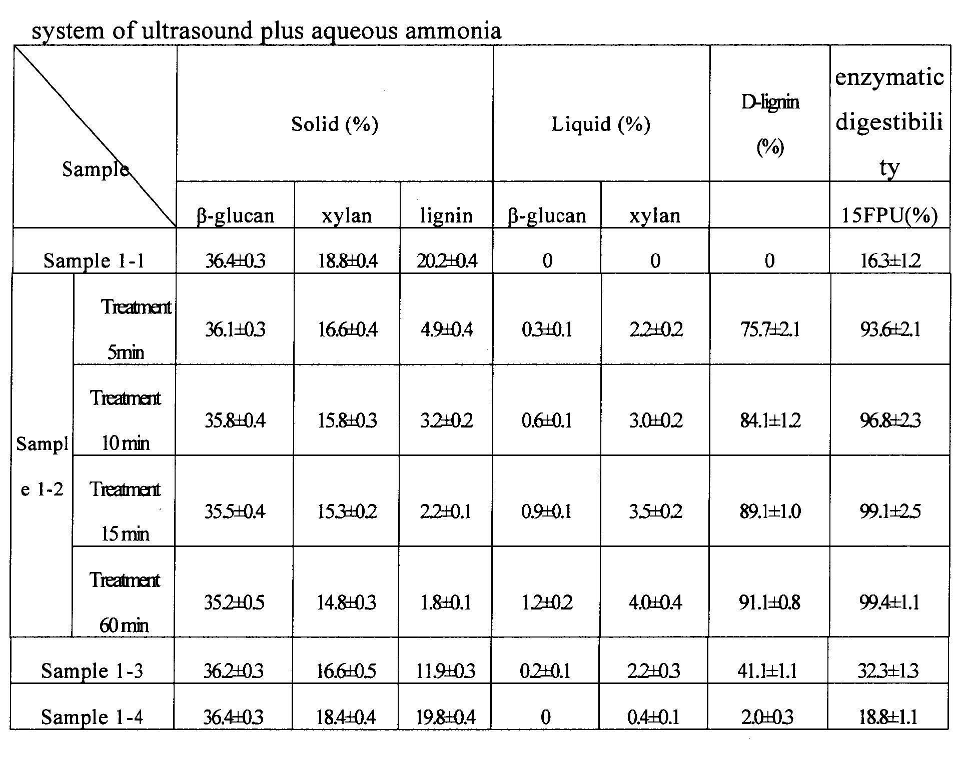

In this table: D-lignin% refers to percentage of lignin removed from the solid sample.

The result shows that ultrasound can remove more than 90% of lignin when aqueous ammonia is used as solvent, and 10 minutes is enough to achieve an enzymatic digestibility of more than 96%. In contrast, the sample 1-4 which was not treated by ultrasound, the sample 1-3 which does not use aqueous ammonia as solvent, and the sample 1-1 which is not treated at all had extremely low efficiency.

### Example 2: Influence of the microwave pretreatment time on the pretreatment effect

7.0 g of air dried, milled maize straw were added into a suitable container (such as a PFA or PTFE reactor, PTFE-polytetrafluoroethylene; PFA-perfluoralkoxy), and then 100 ml aqueous ammonia of 15wt% was added as solvent. The container was closed and sealed appropriately and then treated by microwave in a 1000W/2.45GHz microwave reactor with the container comprising the maize straw continually shaken during the treatment. Sample obtained in this way was Sample 2-2. Samples 2-3, 2-4 and 2-1 were used in comparative experiments. Sample 2-3 was prepared by using deionized water as solvent and treatment by a 1000W/2.45GHz microwave for 00 minutes; sample 2-4 was prepared by using aqueous ammonia as solvent and no microwave treatment; sample 2-1 was not subjected to any treatment. After treatment, the samples were allowed to cool down to ambient temperature, then about 10ml liquid sample was taken from the container comprising the maize straw and used for analysis, the other liquid sample treated by microwave was pooled together and extracted with toluene (2 × 200 ml). The solid sample was washed with deionized water to remove aqueous ammonia, dried under deep freeze and vacuum, and then sampled for solid composition analysis and enzymatic digestibility analysis. The results are shown in Table 2.

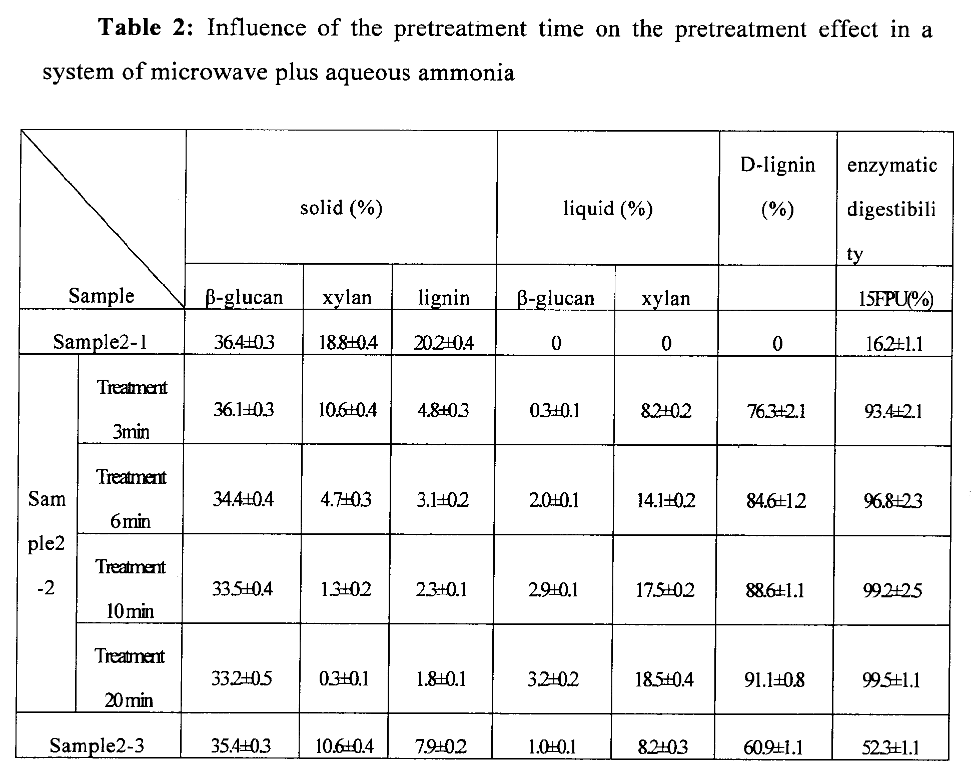

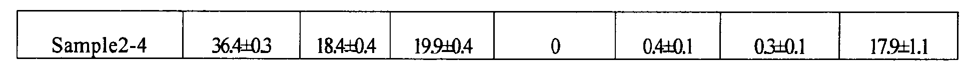

The result shows that the system of microwave and aqueous ammonia has better effect in terms of enzymatic digestibility and lignin removal in a short time period. However, this system lose too much xylan, and the temperature in this system rises too rapidly.

### Example 3A: Influence of different catalysts on the pretreatment effect

20.0 g of air dried, milled maize straw were added into a suitable container, and then 300 ml of sodium hydroxide, potassium hydroxide, hydrochloric acid and diluted sulfuric acid of 1N was added respectively to prepare samples 3A-1, 3A-2, 3A-3, and 3A-4. The container was closed and sealed appropriately and then treated by ultrasound in a 2kW/20kHz ultrasound reactor for 15 minutes with the container comprising the maize straw continually shaken during the treatment. After treatment, about 10ml liquid sample was taken from the container comprising the maize straw and used for analysis, the other liquid sample was pooled together and extracted with toluene (2 × 200 ml). The results are shown in Table 3A.

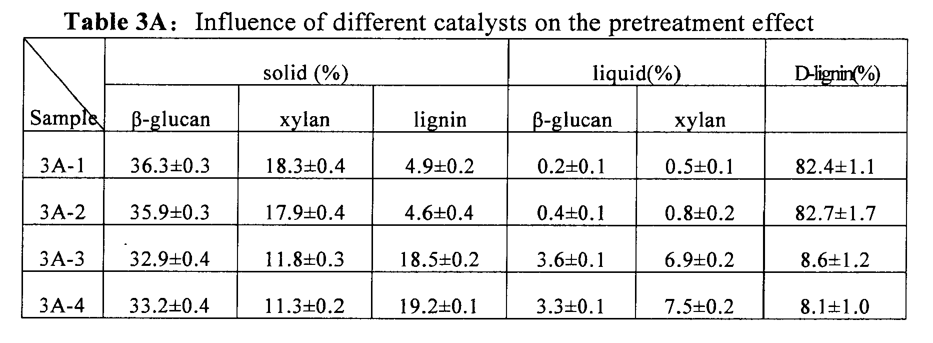

### Example 3B: pretreatment effect of various biomass

20 g of air dried, milled biomass, namely, wheat straw, soybean straw, and cotton straw, respectively, were added into a suitable container, and then 300 ml aqueous ammonia of 15wt% were added. The container was closed and sealed appropriately and then treated by ultrasound in a 2kW/20kHz ultrasound reactor with the container comprising the biomass continually shaken during the treatment. After treatment, about 10ml liquid sample was taken from the container comprising the biomass and used for analysis, the other liquid sample was pooled together and extracted with toluene (2×200 ml). The solid sample was washed with deionized water to remove aqueous ammonia, dried under deep freeze and vacuum, and then sampled for solid composition analysis and enzymatic digestibility analysis. The results are shown in Table 3B.

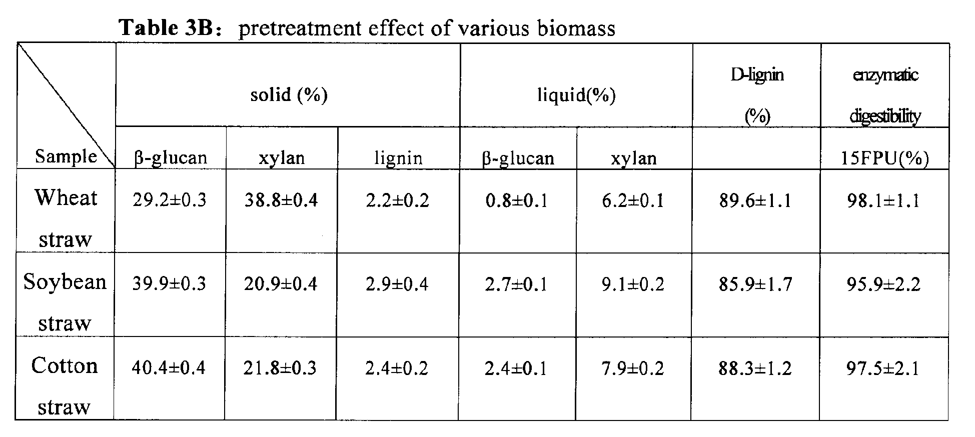

### Example 3: Influence of different liquid volumes on the pretreatment effect

20.0 g of air dried, milled maize straw were added into a suitable container, and then 20, 40, 60, 80, 100, 200, 300 ml aqueous ammonia of 15wt% was added, respectively, to prepare sample 3-1, 3-2, 3-3, 3-4, 3-5, 3-6, and 3-7. The container was closed and sealed appropriately and then treated in a 2kW/20kHz ultrasound reactor with the container comprising the maize straw continually shaken during the treatment. After treatment, about 10ml liquid sample was taken from the container comprising the maize straw and used for analysis, the other liquid sample was pooled together and extracted with toluene (2×200 ml). The solid sample was washed with deionized water to remove aqueous ammonia, dried under deep freeze and vacuum, and then sampled for solid composition analysis and enzymatic digestibility analysis. The results are shown in Table 3.

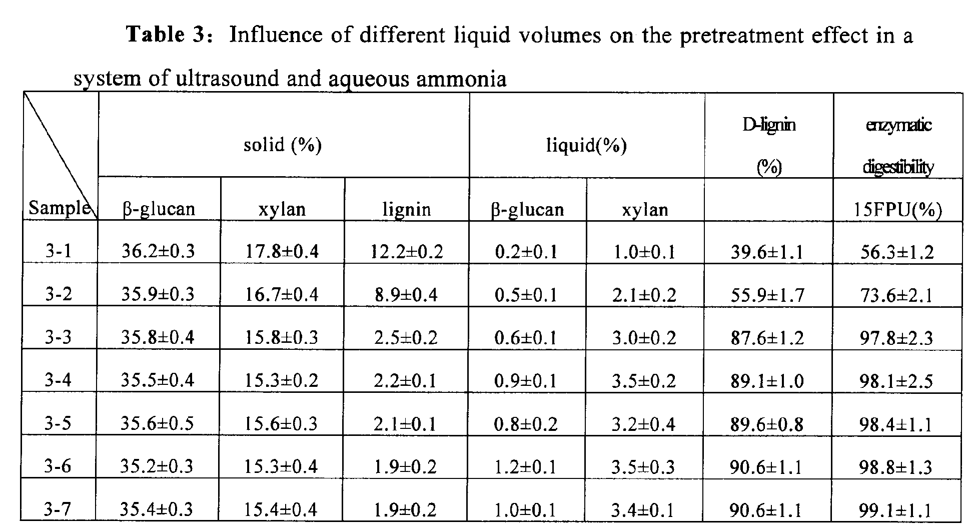

The result shows that the enzymatic digestibility and lignin removal after treatment of sample 3-3 (solid/liquid ratio=1:3, i.e. wt:v, ratio of weight vs. volume) is comparable with that of sample 3-7 (solid/liquid ratio=1: 15) when the biomass is treated with a system of ultrasound and aqueous ammonia. Therefore, the liquid volume used can be as low as solid/liquid ratio=1:3 (wt:v) .

### Example 4: influence of incubation with lime (calcium hydrate) during storage of biomass on the pretreatment effect

Air-dried maize straw and the lime of about 10wt% of the maize straw were mixed thoroughly and stored for 10 days, then the lime was removed by shaking, and the remaining maize straw was milled into particles of 1.6-2.4mm. 20.0 g of air dried, milled maize straw were added into a suitable container, and then 200 ml aqueous ammonia of 15wt% was added. The container was closed and sealed appropriately and then treated in a 2kW/20kHz ultrasound reactor with the container comprising the maize straw continually shaken during the treatment. After treatment, about 10ml liquid sample was taken from the container comprising the maize straw and used for analysis, the other liquid sample was pooled together and extracted with toluene (2×100 ml). The solid sample was washed with deionized water to remove aqueous ammonia, dried under deep freeze and vacuum, and then sampled for solid composition analysis and enzymatic digestibility analysis. The results are shown in Table 4.

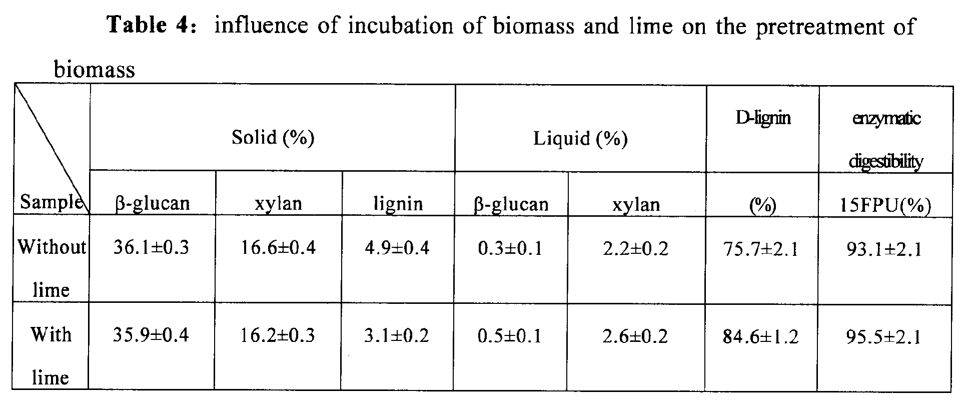

The result shows that when biomass is pretreated with a system of ultrasound and aqueous ammonia, incubation with lime increases the pretreatment effect of biomass in terms of enzymatic digestibility and lignin removal, in comparison with pretreatment of biomass that has been not stored together with lime. Lime can compress biomass, and thus may be used to increase density of biomass, thereby reducing its volume and facilitating its storage and transportation.

### Example 5: Effect of lignin removal and Simultaneous Saccharification and Fermentation

20.0 g of air dried, milled maize straw were added into a suitable container, and then 1000 ml aqueous ammonia of 15wt% was added. The container was sealed appropriately and then treated in a 2kW/20kHz ultrasound reactor for 30 minutes with the container comprising the maize straw continually shaken during the treatment. After treatment, liquid sample was taken from the container comprising the maize straw and used for analysis, the other liquid sample was extracted with tetrahydronaphthalene (THNP, bp=207°C) (2×100 ml) for the measurement of lignin content in the water solution. The solid sample was washed with deionized water to remove aqueous ammonia, vacuum dried, and measured for ethanol conversion by the method of Simultaneous Saccharification and Fermentation (NREL standard laboratory method). Xylan content in the reaction system was 3wt% (weight/volume). The results are shown in Table 5.

**Table 5: Effect of lignin extraction**

| Liquid product | | | | Result of Simultaneous Saccharification and Fermentation |
|---|---|---|---|---|
| β-glucan(%) | xylan(%) | Before extraction lignin(%) | After extraction lignin(%) | After 24 hours, the concentration of ethanol was 14.8g/L(calculated value: 86%) |
| 0.9±0.1 | 3.5±0.2 | 18.0±1.1 | 0.9±0.1 | |

The result shows that the extraction rate of lignin can up to be 95% when tetrahydronaphthalene is used. The pretreated solid biomass can readily be converted into ethanol product.

### Example 6: conversion of lignin into gasoline product

Most toluene in the lignin toluene solution obtained in examples 1-4 was removed under reduced pressure to give a viscous product (substantially lignin) . Meanwhile, most moisture in the product was also removed by azeotrope with toluene. To a Hastolly C pressure reactor were added 20g lignin viscous product, 20g tetrahydronaphthalene and 40g zinc chloride. The mixture was then heated to a temperature of 390-400°C under a pressure of 12Mpa and retained at that temperature for 1 hour. The reaction was magnetically agitated. After the reaction was over, it was allowed to cool down to the ambient temperature. Analysis of the liquid composition reveals that the reaction generates 8.4% water, and the C₆-C₁₂ components with a boiling point between 60-200°C accounts for 86.8% (gasoline). The result shows that lignin obtained by the biomass pretreatment process according to the present invention is good raw material for the production of gasoline.

All the documents cited herein are incorporated into the invention by reference, as if each of them is individually incorporated. Further, it would be appreciated that, in the above teachings of the invention, the skilled in the art could make various changes or modifications to the invention, and these equivalents would still be within the scope of the invention defined by the appended claims of the application.

## Claims

1. A method for the pretreatment of biomass, comprising the steps of:
a) providing milled biomass;
b) mixing the biomass obtained in step a) and the 0.001wt%-50wt% aqueous solution of catalyst to make a mixture of the biomass and catalyst, wherein the weight versus volume ratio of the biomass and the aqueous solution of catalyst is 1:1-1000;
wherein said catalyst is selected from a water soluble alkali compound or a water soluble diluted acid compound;
c) treating the mixture obtained in step b) by using a force field at ambient temperature and under ambient pressure for a time period ranging from 1 second to 1000 minutes, then isolating the treated mixture by filtration to give the pretreatment product of the biomass, wherein said pretreatment product includes liquid and solid portions.

2. The method of claim 1, wherein the catalyst in step b) is aqueous ammonia of 0.001wt%-30wt%, the weight versus volume ratio of the biomass and the aqueous ammonia is 1:1-1000.

3. The method of claim 2, wherein the weight versus volume ratio of the biomass and the aqueous ammonia is 1:2 -20, more preferably 1:3-10.

4. The method of claim 1, wherein the force field is microwave, ultrasound, or the combination thereof.

5. The method of claim 4, wherein frequency of the microwave is in the range of 300 megahertz (MHz) to 300gigahertz (GHz), intensity of the microwave is in the range of 0.1 W to 10kW,
Or frequency of the ultrasound is in the range of 17kHz to 300MHz, intensity of the ultrasound is in the range of 0.1 Watts/cm² to 300 Watts/cm².

6. The method of claim 1, wherein the duration time of step c) is between the range from 1 minute to 60 minutes

7. The method of claim 1, wherein the biomass is placed together with lime in a ratio of 1∼ 100wt% for a time period of 1 day to 180 days in step a).

8. A method of producing liquid fuel from biomass, comprising the steps of:
(i) hydrolyzing the solid portion of the pretreatment product obtained according to claim 1 with enzymatic catalyst to obtain monosaccharide, which is then fermented and purified to give the first liquid fuel; and/or
(ii) isolating the liquid portion of the pretreatment product obtained according to claim 1 to obtain lignin, which is then hydrogenized to give the second liquid fuel.

9. The method of claim 8, wherein the method of isolating the liquid product in step (ii) is extracting the liquid product with polar aromatic compound, said polar aromatic compound is selected from the group consisting of mono-substituted benzene, poly-substituted benzene, aqueous naphthalene derivative, aqueous phenanthrene derivative, aqueous anthracene derivative, or the combination thereof.

10. The method of claim 8, wherein the catalyst collected from washing of the solid product with water or hot steam in step (i) and the catalyst left after extraction of lignin from the liquid product in step (ii), upon adjustment of the concentration, is used in step b).

11. Use of biomass for the simultaneous production of ethanol liquid fuel and gasoline liquid fuel.
